# EUROPÄISCHE PATENTSCHRIFT

(11) **EP 0 967 202 B1**
(45) Veröffentlichungstag und Bekanntmachung des Hinweises auf die Patenterteilung: **07.05.2003**
(21) Anmeldenummer: 99111626.0
(22) Anmeldetag: 16.06.1999
(51) Int. Cl.: C07C 401/00, B01J 19/12

(54) **Photolyse von 7-Dehydrocholesterin**
Photolysis of 7-dehydrocholesterol
Photolyse du 7-dehydrocholesterol

(30) Priorität: 23.06.1998 EP 98111492
(43) Veröffentlichungstag der Anmeldung: 29.12.1999
(73) Patentinhaber: F. HOFFMANN-LA ROCHE AG, 4070 Basel (CH)
(72) Erfinder: Jansen, Michael, 68870 Bartenheim (FR); Braun, André M., 76889 Barbelroth (DE); Troscher, Gabriele, 76137 Karlsruhe (DE); Popp, Hanns-Peter, 82335 Berg (DE)
(74) Vertreter: Buntz, Gerhard

(56) Entgegenhaltungen:
- EP-A- 0 118 903
- EP-A- 0 697 374
- DD-A- 279 668
- DE-A- 4 136 949
- US-A- 4 388 242
- US-A- 4 686 023
- CHEMICAL ABSTRACTS, vol. 114, no. 9, 4. März 1991 (1991-03-04) Columbus, Ohio, US; abstract no. 82251, IKEDA, MASAHIRO ET AL.: "Laser photochemical synthesis of vitamin D analog" XP002116899 & REZA KAGAKU KENKYU, Bd. 11, 1989, Seiten 24-27, Jp
- DATABASE COMPENDEX [Online] ENGINEERING INFORMATION, INC., NEW YORK, NY, US SHANGGUAN CHENG ET AL: "Research of impure 7-DHC irradiated by UV laser beams" Database accession no. EIX93091650168 XP002116900 & YINYONG JIGUANG;YINYONG JIGUANG/APPLIED LASER TECHNOLOGY FEB 1993 CHINA INT BOOK TRADING CORP, BEIJING, CHINA, Bd. 13, Nr. 1, Februar 1993 (1993-02), Seiten 29-31,

## Beschreibung

Die Erfindung betrifft ein photochemisches Verfahren zur Herstellung von gegebenenfalls hydroxylierten oder acylierten Prävitamin D₃ aus 7-Dehydrocholesterin in einem besonderen Reaktor mit einer UV-Strahlungsquelle.

Es ist bekannt, dass Prävitamin D₃ aus 7-Dehydrocholesterin (7-DHC) durch Bestrahlung zu erhalten ist. Dieses Prävitamin lässt sich durch thermische Umlagerung in das thermisch stabilere Vitamin D₃ überführen.

Die photochemische Synthese des Prävitamins erfolgte bisher in technischem Massstab durch Bestrahlung von 7-DHC mittels Quecksilber-Mitteldruck-Lampen. Da Ausgangsprodukt, Primärprodukt, aber auch Sekundärprodukte, im gleichen Wellenlängenbereich mit unterschiedlicher Effizienz absorbieren, wird mit polychromatischer Strahlung, wie sie von diesen Lampen geliefert wird, die Bildung photochemischer Sekundärprodukte begünstigt, die unwirksam und zum Teil toxisch sind. Beim derzeitigen Stand der Technik ist es daher erforderlich, die Bestrahlung schon nach relativ geringem Umsatz abzubrechen. Das nicht umgesetzte 7-DHC wird rückgeführt, während das Primärprodukt in einem aufwendigen Aufbereitungsprozess gereinigt werden muss.

Eine weitere Konsequenz der im gleichen Wellenlängenbereich absorbierenden Substrate und Produkte sind Filtereffekte. So überlappt z.B. das Absorptionsspektrum von Prävitamin vollständig mit dem von 7-DHC, und das Prävitamin absorbiert mit zunehmendem Umsatz einen ständig wachsenden Anteil des Lichts.

Eine weitere Ursache für den Abbruch der Reaktion bei relativ geringem Umsatz (10-20%) ist die Tatsache, dass die Quantenausbeute der photochemischen Folgereaktion von Prävitamin D₃ z.B. zu Tachysterol grösser ist als die Quantenausbeute seiner Bildung.

Wichtigstes Problem bei der derzeitigen Produktion des Prävitamins D₃ ist aber die schlechte Abstimmung des Emissionsspektrums der Quecksilber-Mitteldruck-Lampen auf das Absorptionsspektrum von 7-DHC. Dies führt dazu, dass nur etwa 1% der Strahlung einer Quecksilber-Mitteldruck-Lampe im gewünschten Bereich zwischen 280 und 300 nm nutzbar sind. Diese Abschätzung vernachlässigt ausserdem den nicht unerheblichen Anteil der Produktion von unerwünschten Nebenprodukten durch Bestrahlung ausserhalb dieses optimalen Wellenlängenbereiches.

Der Erfindung liegt die Aufgabe zugrunde, ein photochemisches Verfahren zur Herstellung von gegebenenfalls hydroxyliertem oder acyliertem Prävitamin D₃ aus 7-Dehydrocholesterin in einem Reaktor mit einer UV-Strahlungsquelle zur Verfügung zu stellen, das weniger Nachteile als die Verfahren nach dem Stand der Technik aufweist.

Diese Aufgabe wird erfindungsgemäss dadurch gelöst, dass es sich bei dem Reaktor um einen Fallfilmreaktor handelt, und dass die Strahlungsquelle ein mit XeBr gefüllter Excimer- bzw. Exciplexstrahler ist, der nach dem Mechanismus der 'stillen Entladung' im UV-Bereich quasi monochromatisch emittiert.

Als Stand der Technik offenbart EP 0 697 374 A1 eine Vorrichtung zum Behandeln von Fluiden mit UV-Strahlung, welche durch einen Excimerstrahler erzeugt wird. Die Vorrichtung eignet sich für die Durchführung von wellenselektiven Photoreaktionen und kann somit als "Reaktor" bezeichnet werden. Die Vorrichtung beinhaltet im Fluidraum einen statischen Mischer für das Fluid, welcher zugleich als Elektrode dient und eine ganz spezielle Konfiguration aufweist. Diese Elektrode steht während der Bestrahlung des zu behandelnden Fluids nicht nur mit dem durchlaufenden, zu behandelnden Fluid in Kontakt, sondern bewegt und mischt es. Auf diese Weise soll die Vorrichtung eine effizientere Bestrahlungsbehandlung ermöglichen als der in EP 0 245 111 B1 beschriebene Excimerstrahler. Gemeinsam ist allerdings der ständige Kontakt der inneren Elektrode mit dem zu behandelnden Fluid. Im Gegensatz zu diesen bekannten Strahlern/Reaktoren weist der im erfindungsgemässen Verfahren verwendete "Fallfilmreaktor" eine UV-Strahlungsquelle (einen Excimer- oder Exciplexstrahler), welche vom zu bestrahlenden Medium abgetrennt ist: keine der Elektroden steht in Kontakt mit dem Medium. Des Weiteren sind in EP 0 697 374 A1 im Zusammenhang mit der Verwendung der offenbarten Vorrichtung die Excimere bzw. Exciplexe Xe, Kr, Ar, F₂, Cl₂, Br₂, I₂, Xe₂ und XeCl zwar erwähnt, allerdings nur in einem allgemeinen Sinne. Lediglich in Bezug auf die wellenlängenselektive Photolyse von 7-DHC zur Produktion von Vitamin D₃ ist Br₂ als Excimergas spezifisch genannt.

Die Emissionswellenlänge der Excimer- oder Exciplexstrahler wird durch die Zusammensetzung der Excimer- bzw. Exciplex-bildenden Gase bestimmt. Excimer- bzw. Exciplexstrahler haben die besondere Eigenschaft, dass sie in ihrer Geometrie sehr variabel sind und in einem sehr engen Wellenlängenbereich emittieren. Sie können daher entsprechend den erforderlichen Reaktionsbedingungen im Rahmen der verfügbaren Gase und Materialien optimal ausgewählt und konstruiert werden. Die für die Herstellung von Vitamin D₃ erforderliche elektrische Leistung pro kg Produkt wird durch den Einsatz dieser Strahler erheblich verringert. Die monochromatische Strahlung der Excimer- bzw. Exciplexstrahler führt auch zu einer erheblichen Verbesserung der Produktreinheit.

Das Verfahren, Photonen aus Excimer- bzw. Exciplex-Reaktionen zu bilden, ist aus der Lasertechnologie bekannt. So offenbart die US-Patentschrift 4 388 242 die photolytische Umwandlung von 7-DHC in Prävitamin D₃ mittels eines Excimer-Lasers (KrF bei 248 nm); eine anschliessende Anreicherung des in Produktegemisch der ersten Verfahrensstufe befindlichen Prävitamins D₃ wird unter Einsatz von Lichtbestrahlung im Wellenlangebereich 330-360 nm realisiert, wobei als Strahler ein XeF-Laser oder ein XeCl- oder KrF-Excimerstrahler verwendet werden kann. Auch EP 0 118 903 A1 offenbart ein photochemisches Verfahren zur Herstellung von Prävitamin D₃ aus 7-DHC unter Verwendung eines Lasers als Strahlungsquelle, wobei der Laser u.a. ein Excimer - oder Exciplex-Laser sein kann; als Excimer-Laser kommen insbesondere CIF- oder Br₂-Laser, und als Exciplex-Laser insbesondere XeBr-Laser, in Frage. Schliesslich offenbart Reza Kagaku Kenkyu 11, 24 - 7 (1989)/Chem. Abs. 114, No. 9, 82251 (1991) die photochemische Synthese von Vitamin D₃ über Prävitamin D₃ aus 7-DHC unter Verwendung eines KrF-Lasers, gefolgt von einer 400 W-Hg-Lampe. In keinem dieser drei Dokumente ist ein Fallfilmreaktor als Verfahrensreaktor beschrieben oder suggeriert. Laser-Photonenquellen sind wegen des hohen technischen Aufwands, ihrer für die präparative Photochemie wenig geeigneten Strahlungsgeometrie und der damit verbundenen ungenügend grossflächigen Strahlungsdichte für die photochemische Synthese von Prävitamin D₃ nicht geeignet.

Mit den erfindungsgemäss verwendeten Strahlern werden somit erstmals Strahlungsquellen eingesetzt, die praktisch ausschliesslich im optimalen Wellenlängenbereich der photochemischen Prävitamin-D₃-Synthese emittieren und mit denen die oben beschriebenen Nachteile überwunden werden können. Sie eignen sich daher gut als Lichtquellen für die Prävitamin-D₃-Synthese in technischem Massstab.

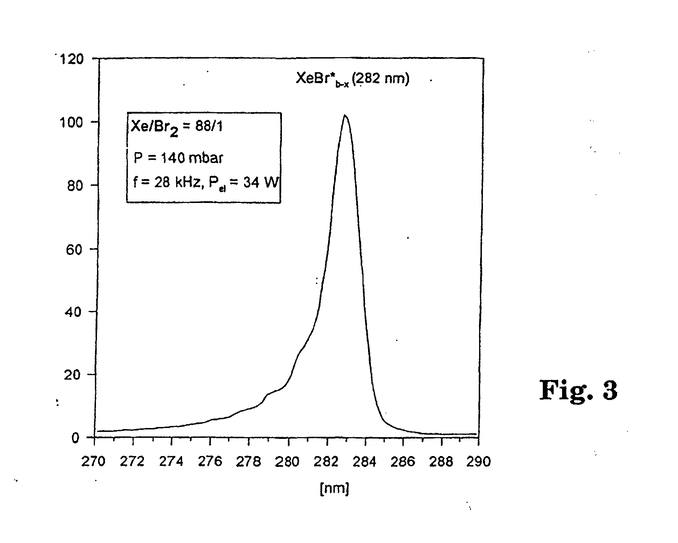

Typische Beispiele für Strahler, deren Emissionswellenlänge in dem für die Prävitamin-D₃-Synthese erforderlichen optimalen Wellenlängenbereich liegt, sind mit XeBr gefüllte Excimerstrahler, die Photonen bei 285 bzw. 292 nm Wellenlänge emittieren. Als Beispiel wird das Emissionsspektrum der XeBr-Lampe in Fig. 3 gezeigt.

Bei den erfindungsgemäss einsetzbaren Strahlern werden Excimere bzw. Exciplexe (XeBr) gebildet, die bei Zerfall Photonen der gewünschten Wellenlänge emittieren. Beide Strahler haben sich bei ihrer Anwendung auf die photochemische Prävitamin-D₃-Synthese als gute und leistungsfähige Strahlungsquellen erwiesen.

Das Verfahren der Erfindung eignet sich in gleicher Weise zur Herstellung der hydroxylierten bzw. acylierten Prävitamin D-Derivate (z.B. 1a-Hydroxy- bzw. 25-Hydroxy oder Acyloxyprävitamin D₃).

Im folgenden wird anhand der beiliegenden Zeichnungen ein Ausführungsbeispiel der Erfindung beschrieben. Es zeigen
Fig. 1 den Aufbau des Fallfilmereaktors
Fig. 2 einen vergrösserten Ausschnitt des Fallfilmreaktors
Fig. 3 das Emissionsspektrum eines geeigneten Strahlers

7-Dehydrocholesterin wurde in 3,5%iger Lösung in einem Methanol/Hexan-Gemisch mit einer XeBr-Excimerlampe bei 284 nm in einem Fallfilmreaktor bestrahlt. In regelmässigen Zeitabständen wurde die Zusammensetzung des Reaktionssystems durch Hochleistungs-Flüssig-Chromatographie bestimmt. Bei dieser Wellenlänge besteht bei einem Umsatz von ca. 50% das erhaltene Reaktionsgemisch zu über 93% aus dem gewünschten Prävitamin D₃. Das Ausgangsmaterial lässt sich nach bekannten Verfahren leicht abtrennen.

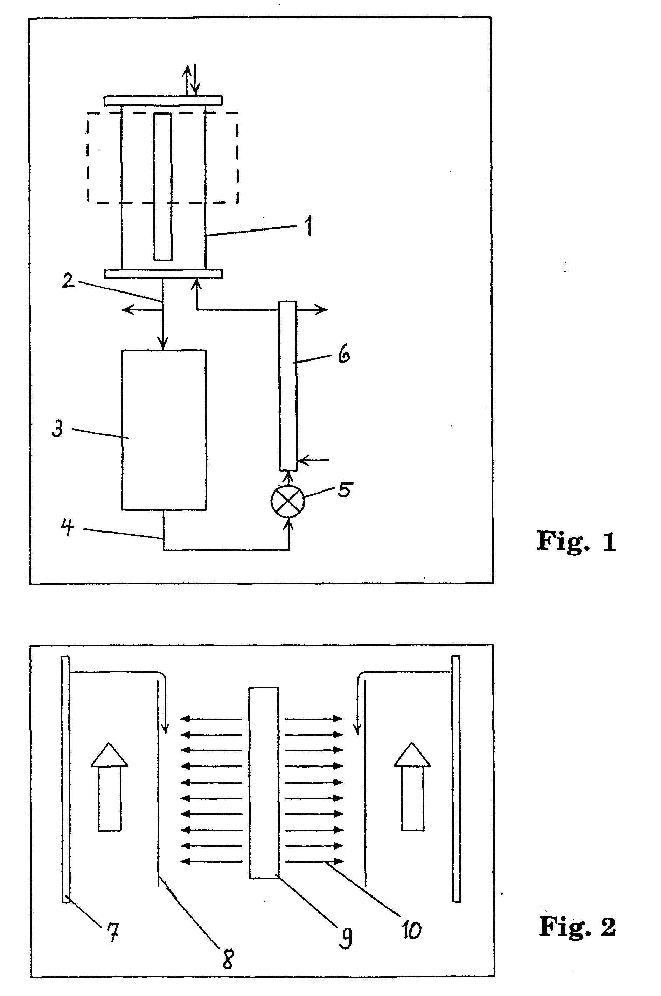

Die in Fig. 1 gezeigte Apparatur besteht aus einem Fallfilmreaktor 1, dessen Austragsleitung 2 direkt mit einem Vorlagebehälter 3 verbunden ist. Der Vorlagebehälter enthält den grössten Teil des Reaktionsgemisches. Eine Zuführungsleitung 4 für das Reaktionsgemisch führt vom Vorlagebehälter über eine Pumpe 5 und einen Wärmetauscher 6 zum Fallfilmreaktor. Der Fallfilmreaktor ist mit Leitungen 6 zur kontinuierlichen Spülung seines Innenraums mit Stickstoff versehen.

Die Austragsleitung 2 ist mit einem Anschluss für eine kontinuierliche Probenahme versehen.

Aus der vergrösserten Ausschnittdarstellung der Fig. 2 ist der Aufbau des Fallfilmreaktors 1 ersichtlich. Er besitzt einen äusseren zylindrischen Mantel 7. Im Inneren ist koaxial ein zylindrisches Fallrohr 8 aus Quarz angeordnet, dessen Durchmesser etwa dem halben Durchmesser des Mantels entspricht. Ebenfalls koaxial ist in der Mitte der Anordnung ein Excimerstrahler 9 plaziert.

Das Reaktionsgemisch gelangt von unten in den Reaktor und wird als Volumenstrom im ringförmigen Raum zwischen Mantel und Fallrohr nach oben geführt. Von dort strömt das Gemisch als Film an der Innenseite des Fallrohrs nach unten. Dieser Film ist der vom Strahler emittierten Strahlung 10 ausgesetzt.

In dem in Fig. 3 gezeigten Emissionsspektrum des in der Apparatur eingesetzten Xenon-Bromid-Excimer-Strahlers ist auf der Abszissenachse die Wellenlänge der emittierten Strahlung in nm und auf der Ordinatenachse die relative Intensität angegeben.

## Patentansprüche

1. Photochemisches Verfahren zur Herstellung von gegebenenfalls hydroxyliertem oder acyliertem Prävitamin D₃ aus 7-Dehydrocholesterin in einem Reaktor mit einer UV- Strahlungsquelle, **dadurch gekennzeichnet, dass** die Strahlungsquelle ein mit XeBr gefüllter Excimer- bzw. Exciplexstrahler ist, der nach dem Mechanismus der ^{'}stillen Entladung im UV-Bereich quasi monochromatisch emittiert.

2. Photochemisches Verfahren nach Anspruch 1, **dadurch gekennzeichnet, dass** das hydroxylierte oder acylierte Prävitamin D₃ 1a-Hydroxy- bzw. 25-Hydroxy- oder Acyloxyprävitamin D₃ ist.

3. Photochemisches Verfahren nach Anspruch 1 oder 2, **dadurch gekennzeichnet, dass** es sich bei dem Reaktor un einen Fallfilmreaktor handett.

## Claims

1. A photochemical process for the production of optionally hydroxylated or acylated previtamin D₃ from 7-dehydrocholesterol in a reactor having a UV radiation source, **characterized in that** the radiation source is an excimer or exciplex emitter filled with XeBr which emits quasi-monochromatically according to the 'corona discharge' mechanism in the UV range.

2. A photochemical process according to claim 1, **characterized in that** the hydroxylated or acylated previtamin D₃ is 1a-hydroxy- or 25-hydroxy- or acyloxy-previtamin D₃.

3. A photochemical process according to claim 1 or 2, **characterized in that** the reactor is a falling-film reactor.

## Revendications

1. Procédé photochimique de préparation de prévitamine D₃ éventuellement hydroxylée ou acylée à partir de 7-déshydrocholestérol dans un réacteur comportant une source de rayonnement UV, **caractérisé en ce que** la source de rayonnement est un radiateur à excimère ou à exciplexe rempli de XeBr, qui émet d'une manière quasi-monochromatique dans le domaine des UV, par le mécanisme de la décharge silencieuse.

2. Procédé photochimique selon la revendication 1, **caractérisé en ce que** la prévitamine D₃ hydroxylée ou acylée est la 1a-hydroxy- ou la 25-hydroxy- ou l'acyloxy-prévitamine D₃.

3. Procédé photochimique selon la revendication 1 ou 2, **caractérisé en ce que**, pour ce qui concerne le réacteur, il s'agit d'un réacteur à film tombant.
